# EUROPEAN PATENT APPLICATION

(11) **EP 3 020 756 A1**
(43) Date of publication of application: **18.05.2016**
(21) Application number: 14729571.1
(22) Date of filing: 15.04.2014
(51) Int. Cl.: C08K 5/36, C08K 5/13, C08L 21/00, B60C 1/00

(54) **NOVEL CARDANOL-BASED ORGANIC VULCANIZING AGENT, PRODUCTION METHOD FOR SAME AND BLENDED RUBBER COMPOSITION FOR TYRES USING SAME**

(30) Priority: 12.07.2013 KR 20130082035; 08.04.2014 KR 20140041658
(71) Applicant: M&b Greenus Co. Ltd., Yeongju-si, Gyeongsangbuk-do 750-881 (KR)
(72) Inventor: LEE, Hong Dae, Goyang-si Gyeonggi-do 412-748 (KR); KIM, Jin Hwan, Seoul 137-779 (KR); PARK, Je Hwan, Seoul 150-045 (KR); YOON, Joo Young, Hwaseong-si Gyeonggi-do 445-835 (KR); PARK, Ki Un, Suwon-si Gyeonggi-do 441-718 (KR); VO, Thi Hai, Suwon-si Gyeonggi-do 440-828 (KR)
(74) Representative: IP Trust
(86) International application number: PCT/KR2014/003236
(87) International publication number: WO 2015/005568

(57) **Abstract**

Disclosed are a method for preparing a cardanol-based organic vulcanizing agent, including a step of reacting cashew nut extract with powdered sulfur, a cardanol-based organic vulcanizing agent obtained by the method, and a rubber compound and tire composition using the same. The rubber composition using the cardanol-based organic vulcanizing agent shows significantly improved expected mileage (FPS), tensile strength (T/S), non-vulcanized properties and wet traction as compared to the rubber composition using the conventional vulcanizing agent, and contributes to improvement of wear property of tires as final products in actual cars. Therefore, the cardanol-based organic vulcanizing agent can be applied advantageously to rubber compound compositions for tires used in rubber vulcanizing processes for tire treads.

## Description

### [Technical Field]

The present invention relates to a novel cardanol-based organic vulcanizing agent, a method for preparing the same, and a rubber compound composition for tires using the same. More particularly, the present invention relates to a cardanol-based organic vulcanizing agent obtained by reacting cardanol extracted from cashew nuts with powdered sulfur and having excellent rubber processability and rubber compoundability, a method for preparing the same, and a rubber compound composition for tires using the same.

### [Background Art]

When a vulcanizing agent, such as sulfur, metal oxide or organic peroxide, is applied to unsaturated double bonds in chain polymer rubber, the rubber molecules form a kind of reticular connective polymer rubber to provide an elastic body. This is referred to as vulcanization. Some factors of such vulcanization include heat, time, pressure, or the like.

Natural rubber is advantageous in that it can be elongated and has waterproof property, but is disadvantageous in that it becomes sticky upon heating and becomes brittle upon cooling. One method suggested to solve such a problem is a vulcanization method. Since Charles Goodyear found that vulcanization of green rubber with sulfur increases elasticity and solves the problem of stickiness at high temperature and brittleness at low temperature, the vulcanization method has been used essentially throughout the fields of natural rubber and synthetic rubber.

A vulcanizing agent is selected and used adequately depending on the composition, molecular distribution state, structure, etc. of a polymer. In general, vulcanizing agents may be classified into organic vulcanizing agents and inorganic vulcanizing agents. An organic vulcanizing agent is a chemical that performs vulcanization by producing a crosslinked form through the bonding of rubber polymer chains while sulfur contained in its structure is released. Such organic vulcanizing agents include active sulfur-releasing organic vulcanizing agents, multifunctional organic vulcanizing agents including oximes, dinitroso compounds, synthetic resins and polyamines, and peroxide vulcanizing agents.

Sulfur compounds, typical inorganic vulcanizing agents according to the related art, include powdered sulfur obtained by sublimation or melt purification of sulfur of natural acids, insoluble sulfur having a crystalline particle structure and containing insoluble content of carbon disulfide (CS₂), precipitation sulfur obtained by decomposition of multisulfide such as calcium pentasulfide, colloidal sulfur obtained by milling sulfur in a colloid mill or precipitating sulfur from colloidal insoluble solution, or the like. However, such inorganic sulfur compounds are problematic in that they cause degradation of compatibility when used as additives to synthetic resins, and they undergo a drop in adhesion depending on introduction amount. In addition, in terms of compounding properties of rubber for use in tire treads to which inorganic sulfur or the conventional organic vulcanizing agent is applied, there is a limitation in improvement of rolling resistance (R/R), wet traction (T/R), durability (mileage), and wear property.

### [Disclosure]

### [Technical Problem]

A technical problem to be solved by the present invention is to provide a cardanol-based organic vulcanizing agent obtained by reacting organic materials extracted from naturally derived cashew nuts with powdered sulfur, having physical properties and rubber compoundability suitable for rubber processing, and showing eco-friendly characteristics and high cost efficiency, as well as a rubber compound composition for tires using the same.

### [Technical Solution]

In one general aspect, there is provided a method for preparing a cardanol-based organic vulcanizing agent, including a step of reacting cashew nut extract with powdered sulfur.

According to an embodiment, the cashew nut extract may be cardanol.

According to another embodiment, the method includes reacting 100 parts by weight of cashew nut extract with 10-100 parts by weight of powdered sulfur.

According to still another embodiment, the reaction may be carried out at 130-200°C.

According to still another embodiment, the method may further include a step of dissolving the product obtained by reacting cashew nut extract with powdered sulfur into a solvent to perform purification.

According to still another embodiment, the solvent may be at least one selected from the group consisting of ethyl acetate, n-hexane and ethers.

According to still another embodiment, the solvent may be used in an amount of 150-200 parts by weight based on 100 parts by weight of the cardanol-based organic vulcanizing agent.

According to still another embodiment, the cardanol-based organic vulcanizing agent may be represented by the following Chemical Formula 1 or 2: 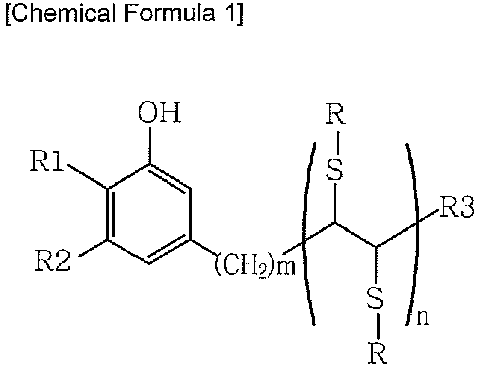 wherein m is an integer of 6 to 13, n is an integer of 1 or 2, R1 is H or methyl, R2 is H or a hydroxyl group, R3 is C_{n'}H_{2n'+1} (wherein n' is an integer of 2 to 5), and R groups are the same or different and each R represents H, S_{n"}H (wherein n" is an integer of 1 to 5) or -S group of 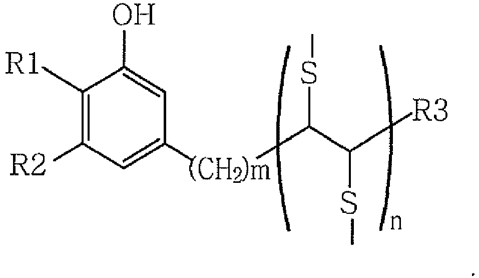 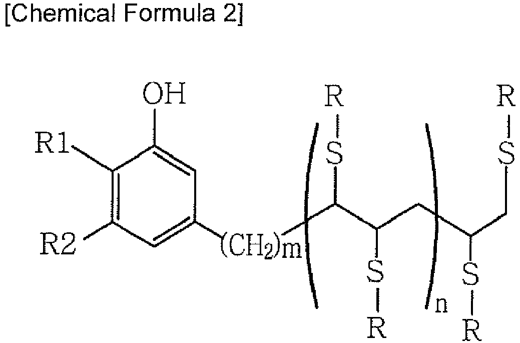 wherein m is an integer of 6 to 13, n is an integer of 1 or 2, R1 is H or methyl, R2 is H or a hydroxyl group, and R groups are the same or different and each R represents H, S_{n"}H (wherein n" is an integer of 1 to 5) or -S group of

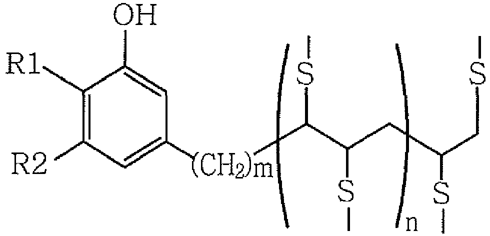

According to yet another embodiment, the cardanol-based organic vulcanizing agent may be represented by the following Chemical Formula 3, 4 or 5: 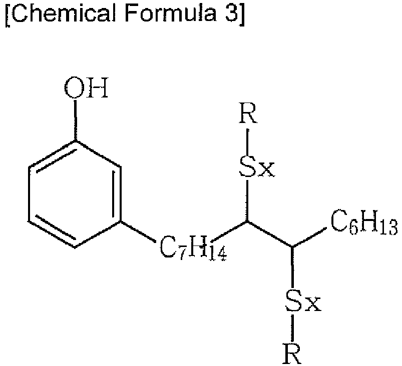 wherein x is an integer of 1 to 4, and R is H. 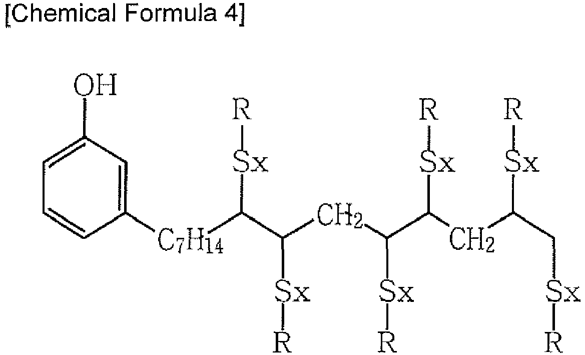 wherein x is an integer of 1 to 4, and R is H. 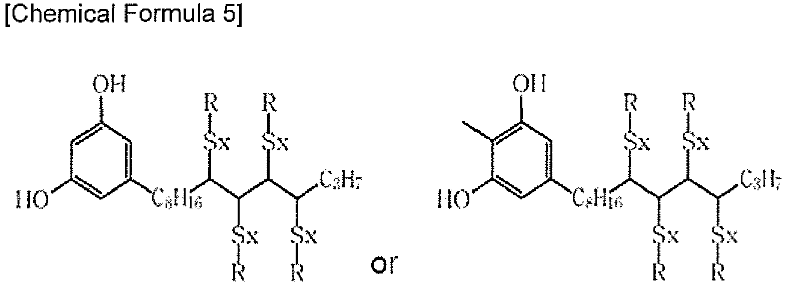 wherein x is an integer of 1 to 4, and R is H.

In another general aspect, there is provided a cardanol-based organic vulcanizing agent obtained by the above method and represented by the above Chemical Formula 1 or 2.

In still another general aspect, there is provided a rubber compound composition including the cardanol-based organic vulcanizing agent.

According to an embodiment, the composition may include 1-2 parts by weight of the cardanol-based organic vulcanizing agent, 50-70 parts by weight of silica and 10-30 parts by weight of carbon black based on 100 parts by weight of rubber as a raw material.

According to another embodiment, the composition may be used for tire treads.

### [Advantageous Effects]

According to the novel cardanol-based organic vulcanizing agent of the present invention, estimated mileage of tires (FPS), tensile strength (T/S), non-vulcanized properties and wet traction are improved significantly as compared to tires using the conventional vulcanizing agents. In addition, the cardanol-based organic vulcanizing agent contributes to improvement of tire wear property in actual cars. Therefore, it can be applied advantageously to rubber compound compositions for tires used in rubber vulcanizing processes for tire treads.

### [Description of Drawings]

Fig. 1 illustrates the results of FT-IR analysis of the cardanol-based organic vulcanizing agent according to an exemplary embodiment and cardanol;
Fig. 2 and Fig. 3 illustrate the results of NMR analysis of the cardanol-based organic vulcanizing agent according to an exemplary embodiment and cardanol; and
Fig. 4 and Fig. 5 illustrate the results of NMR analysis of the cardanol-based organic vulcanizing agent according to an exemplary embodiment and cardanol.

### [Best Mode]

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein.

In one aspect, there is provided a method for preparing a cardanol-based organic vulcanizing agent, including a step of reacting cashew nut extract with powdered sulfur.

According to an embodiment, cardanol, an organic extract extracted from cashew nuts is used as a natural organic material containing an alkyl group having at least one, preferably at least two intramolecular double bonds, instead of a synthetic material such as alkyl phenol as a main ingredient of the conventional organic vulcanizing agent.

There is no particular limitation in the method for extracting cashew nuts, as long as it is any one method generally known to those skilled in the art.

According to an embodiment, the cashew nut extract may be cardanol. Cardanol as extract from cashew nuts contains an alkyl group having a plurality of intramolecular double bonds. After analyzing cardanol by GC/MS, different numbers of double bonds in the alkyl group are found depending on raw material production countries/companies, and different structures have different compositional ratios. For example, two types of cardanol available from different production companies are compared with each other and analyzed. The following shows the main ingredient structures of cardanol analyzed by a GC/MS analyzer. When a compound has one double bond, it is called monoene. When a compound has two or three double bonds, it is called diene or triene.

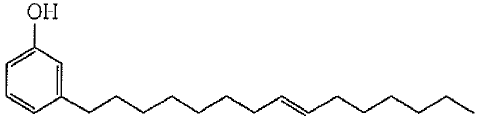

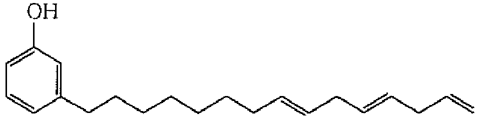

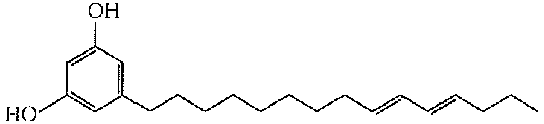

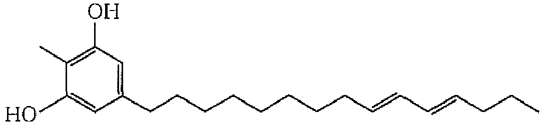

The numbers of double bonds in different types of cardanol available from different production companies are shown in the following Table 1.

**[Table 1]**

| Company | Monoene | Diene | Triene | Sulfur content as synthesized (%) |
|---|---|---|---|---|
| Golden Cashew Products Pvt Ltd. | 67.2% | 20.1% | 11.5% | 30% |
| Biochempia Corp. | 34.6% | 26.7% | 32.3% | 36% |

According to an embodiment, cardanol may include monoene in an amount of 20-70%, preferably 20-50%, and more preferably 20-40% based on (monoene + diene + triene). When the monoene content is larger than the above-defined range, wear property may be degraded.

According to another embodiment, cardanol may include diene + triene in an amount of 20-70%, preferably 30-70%, and more preferably 50-65% based on (monoene + diene + triene). A higher ratio of diene + triene in cardanol is preferred.

The method for preparing a cardanol-based organic vulcanizing agent according to the present invention includes reacting 100 parts by weight of cashew nut extract with 10-100 parts by weight, preferably 20-40 parts by weight of powdered sulfur. When cardanol extracted from cashew nuts is reacted with powdered sulfur within the above-defined range, it is possible to obtain the most effective physical properties for a vulcanization process of rubber for tire treads.

Powdered sulfur may be introduced to cardanol extracted from cashew nuts in portions sequentially or at once, preferably in portions. Herein, powdered sulfur may be introduced preferably considering a degree of heating.

The reaction may be carried out at 130-200°C, preferably 130-180°C, and more preferably 150-180°C. When the reaction temperature is lower than 130°C, powdered sulfur cannot be molten completely, resulting in degradation of its reactivity with cardanol extracted from cashew nuts. When the reaction temperature is higher than 200°C, the reaction temperature may be decomposed again and the product having a desired structure cannot be obtained.

According to still another embodiment, the method may further include a step of dissolving the product obtained from the reaction of cashew nut extract with powdered sulfur into a solvent to perform purification. In this manner, it is possible to improve the purity of the resultant vulcanizing agent.

There is no particular limitation in the solvent used herein. The solvent may be at least one selected from the group consisting of ethyl acetate, n-hexane and ethers, preferably ethyl acetate.

In this case, 100 parts by weight of cardanol extracted from cashew nuts is allowed to react with 10-100 parts by weight of powdered sulfur to provide a cardanol-based organic vulcanizing agent. Then, 100 parts by weight of the non-purified cardanol-based organic vulcanizing agent is dissolved and purified with 150-200 parts by weight of a solvent to obtain a purified cardanol-based organic vulcanizing agent. Preferably, 30-40 parts by weight of cardanol extracted from cashew nuts, 20-30 parts by weight of powdered sulfur and 150-200 parts by weight of a solvent may be used. When using cardanol extracted from cashew nuts, powdered sulfur and a solvent are used within the above-defined range, it is possible to obtain the most effective physical properties for a vulcanization process of rubber for tire treads.

The modified organic vulcanizing agent obtained according to the present invention may be represented by the following Chemical Formula 1 or 2: 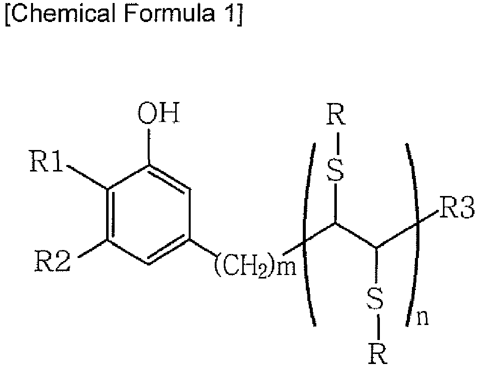 wherein m is an integer of 6 to 13, n is an integer of 1 or 2, R1 is H or methyl, R2 is H or a hydroxyl group, R3 is C_{n'}H_{2n'+1} (wherein n' is an integer of 2 to 5), and R groups are the same or different and each R represents H, Sₙ"H (wherein n" is an integer of 1 to 5) or -S group of 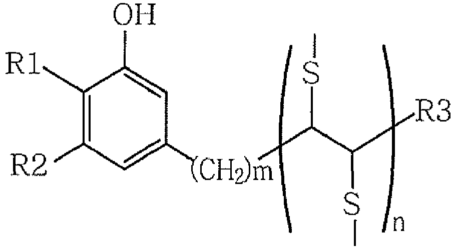 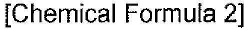 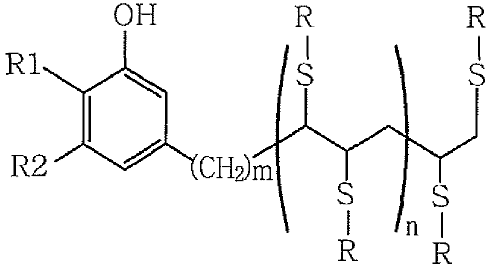 wherein m is an integer of 6 to 13, n is an integer of 1 or 2, R1 is H or methyl, R2 is H or a hydroxyl group, and R groups are the same or different and each R represents H, S_{n"}H (wherein n" is an integer of 1 to 5) or -S group of

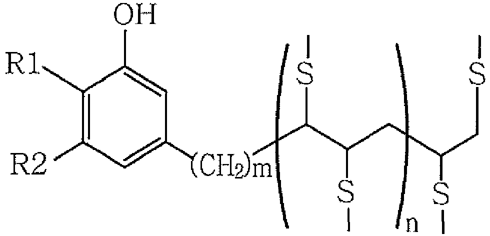

More particularly, the organic vulcanizing agent may be represented by the following Chemical Formula 3, 4 or 5: 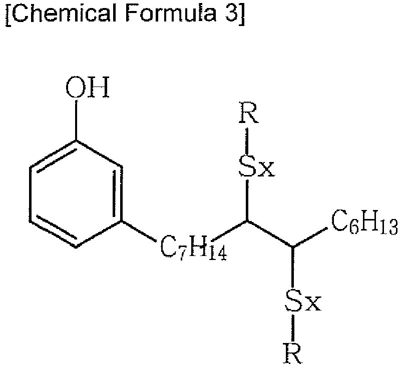 wherein x is an integer of 1 to 4, and R is H. 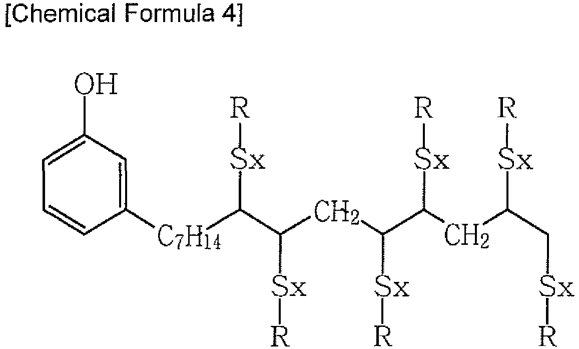 wherein x is an integer of 1 to 4, and R is H. 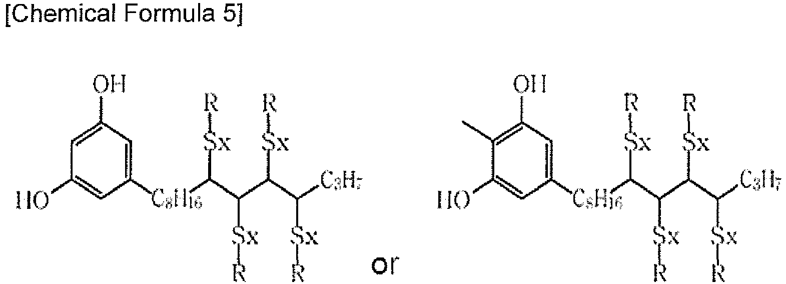 wherein x is an integer of 1 to 4, and R is H.

The method according to the present invention may be represented by the following Reaction Scheme: 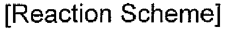 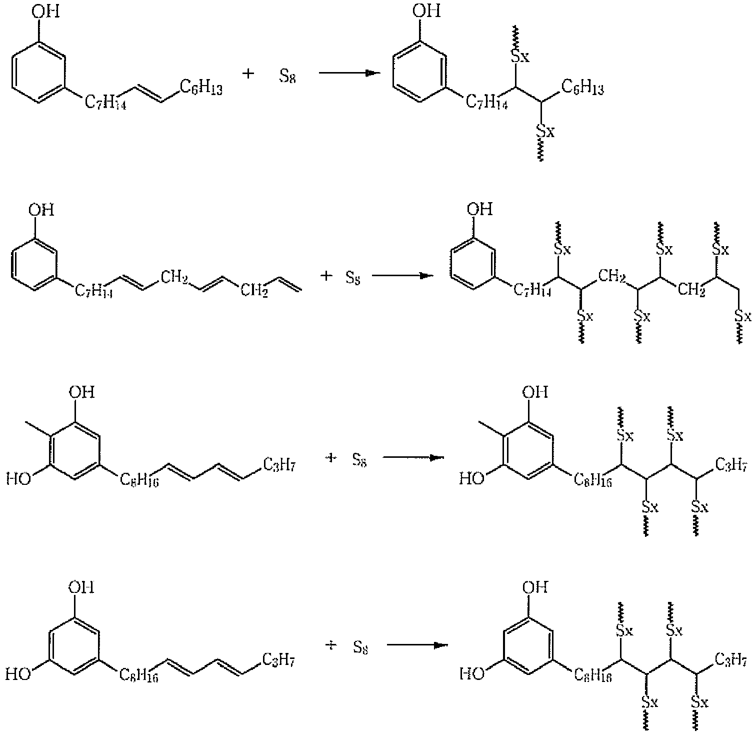 wherein x is an integer of 1 to 4.

The novel cardanol-based organic vulcanizing agent obtained by the method according to the present invention has a high sulfur content, and thus it is possible to improve the problem of a low sulfur occurring in the conventional organic vulcanizing agent using an alkyl phenol resin. In addition, the novel cardanol-based organic vulcanizing agent is eco-friendly because it is derived from natural materials. Further, it shows high cost-efficiency because the production cost can be reduced significantly by virtue of the low cost of cardanol itself. Moreover, a rubber composition using the novel organic vulcanizing agent shows tensile strength and non-vulcanized properties equal to or higher than those of a rubber composition using the conventional organic vulcanizing agent. Particularly, tires obtained by using the rubber composition provides an excellent expected mileage (FPS) as determined by Test Example 1 described hereinafter.

In still another aspect, there is provided a rubber compound composition including the cardanol-based organic vulcanizing agent.

The rubber compound composition according to the present invention may include 1-2 parts by weight of the cardanol-based organic vulcanizing agent, 50-70 parts by weight of silica and 10-30 parts by weight of carbon black based on 100 parts by weight of rubber as a raw material.

There is no particular limitation in the rubber as a raw material, as long as it is one used currently in the art.

The rubber compound composition according to the present invention may include a conventional vulcanizing agent in combination with the above-described cardanol-based organic vulcanizing agent.

Since the rubber compound composition according to the present invention includes the novel cardanol-based organic vulcanizing agent having excellent physical properties, it shows excellent mileage and wet traction property, and thus can be used advantageously for a composition for tire treads.

Examples now will be described more fully hereinafter. The following Examples are for illustrative purposes only and provided to help those skilled in the art to understand the present invention. However, the scope of the present invention should not be construed as limited to the following Examples.

### [Example 1] Preparation of Novel Cardanol-Based Organic Vulcanizing Agent

To a 2L four-necked reaction container equipped with a reactor, agitator, stirring rod, temperature controller and a reflux condenser, 240 g of cardanol available from Golden Cashew Products Pvt. Ltd. as shown in the above Table 1 is introduced and warmed to 140-150°C. Then, 204.8 g of inorganic sulfur is introduced thereto to perform reaction. Herein, sulfur is introduced in portions while maintaining the exothermic temperature at 180°C or lower after observing the heating degree. After the completion of the introduction of inorganic sulfur, the reaction mixture is agitated for 6 hours. After the completion of the reaction, the resultant product is dissolved into ethyl acetate solvent to remove the unreacted materials in the outer wall of reactor and reaction mixture, and the unreacted materials are removed by filtering. The resultant modified organic vulcanizing and ethyl acetate are separated from each other by using an evaporator. Then, the separated cardanol-based organic vulcanizing agent is dried sufficiently in an electric oven.

The cardanol-based organic vulcanizing agent is shown to have 30.1% of sulfur, 0.8% of volatile content and 0% of ash. The cardanol-based organic vulcanizing agent and cardanol are subjected to FT-IR and NMR analysis to determine the reaction degree.

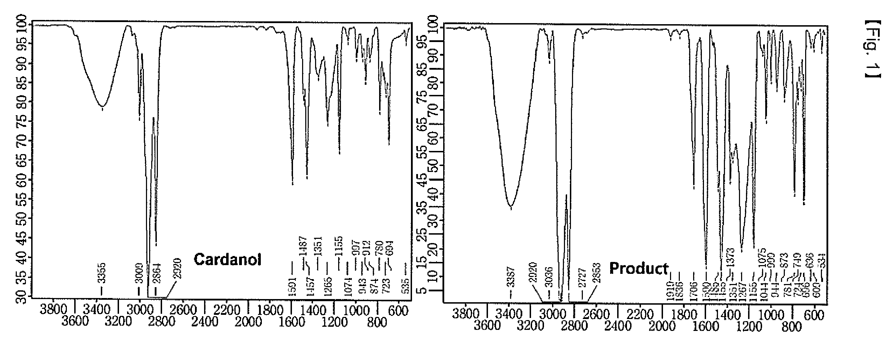

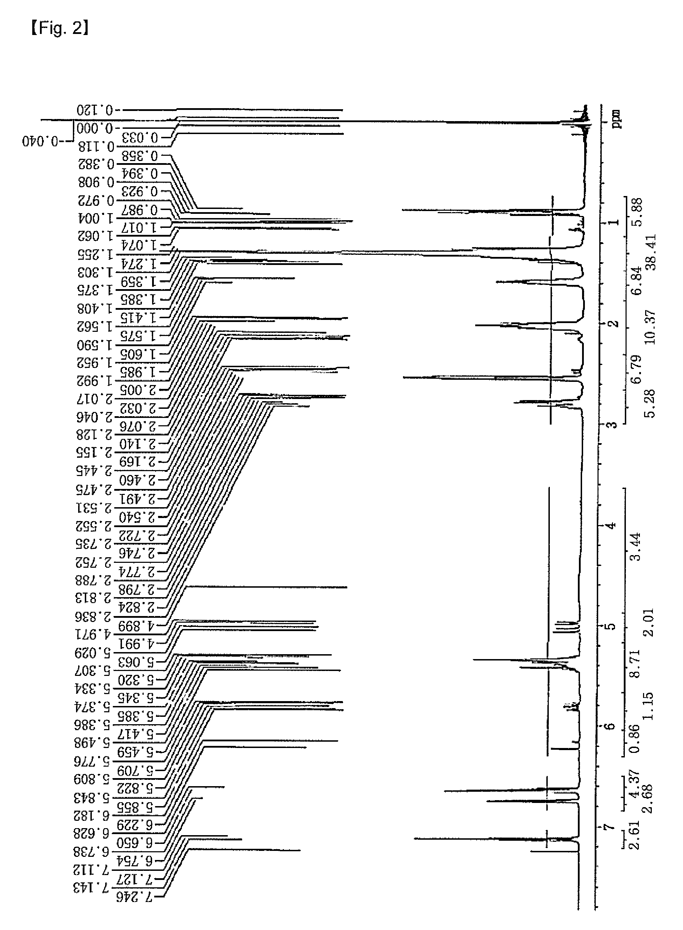

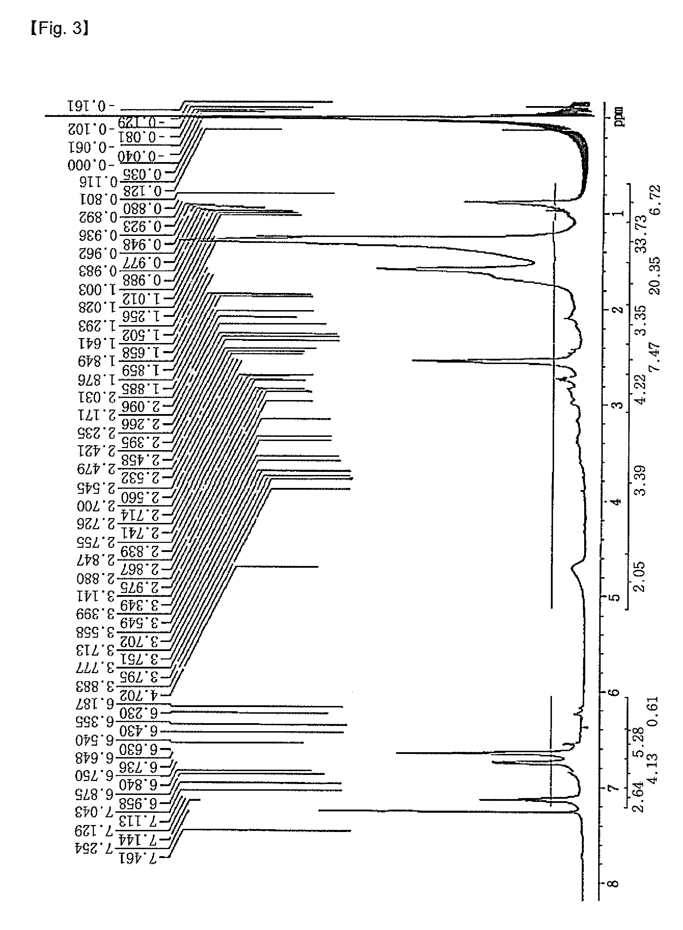

Fig. 1 illustrates the results of FT-IR analysis of cardanol and the cardanol-based organic vulcanizing agent. Fig. 2 and 3 illustrate the results of NMR analysis of cardanol and the cardanol-based organic vulcanizing agent, respectively.

In Fig. 1, the peak appearing at 609 cm⁻¹ (doublet) corresponds to C-S produced by the reaction, and exists only in the product not in cardanol. As shown in Fig. 2, a peak exists at 4.9-5.9 ppm due to double bonds. However, it can be seen from Fig.3 that the double bond peak at 4.9-5.9 ppm disappears.

### [Example 2] Preparation of Novel Cardanol-Based Organic Vulcanizing Agent

To a 2L four-necked reaction container equipped with a reactor, agitator, stirring rod, temperature controller and a reflux condenser, 240 g of cardanol available from Biochem Corp. as shown in the above Table 1 is introduced and warmed to 140-150°C. Then, 204.8 g of inorganic sulfur is introduced thereto to perform reaction. Herein, sulfur is introduced in portions while maintaining the exothermic temperature at 180°C or lower after observing the heating degree. After the completion of the introduction of inorganic sulfur, the reaction mixture is agitated for 6 hours. After the completion of the reaction, the resultant product is dissolved into ethyl acetate solvent to remove the unreacted materials in the outer wall of reactor and reaction mixture, and the unreacted materials are removed by filtering. The resultant modified organic vulcanizing and ethyl acetate are separated from each other by using an evaporator. Then, the separated cardanol-based organic vulcanizing agent is dried sufficiently in an electric oven.

The cardanol-based organic vulcanizing agent is shown to have 36.2% of sulfur, 0.9% of volatile content and 0% of ash.

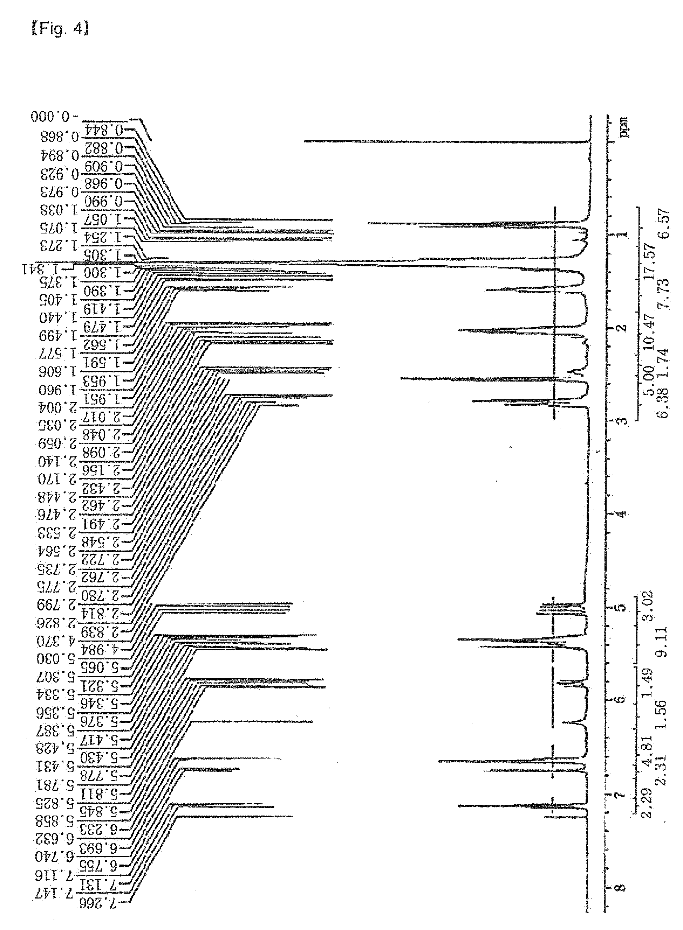

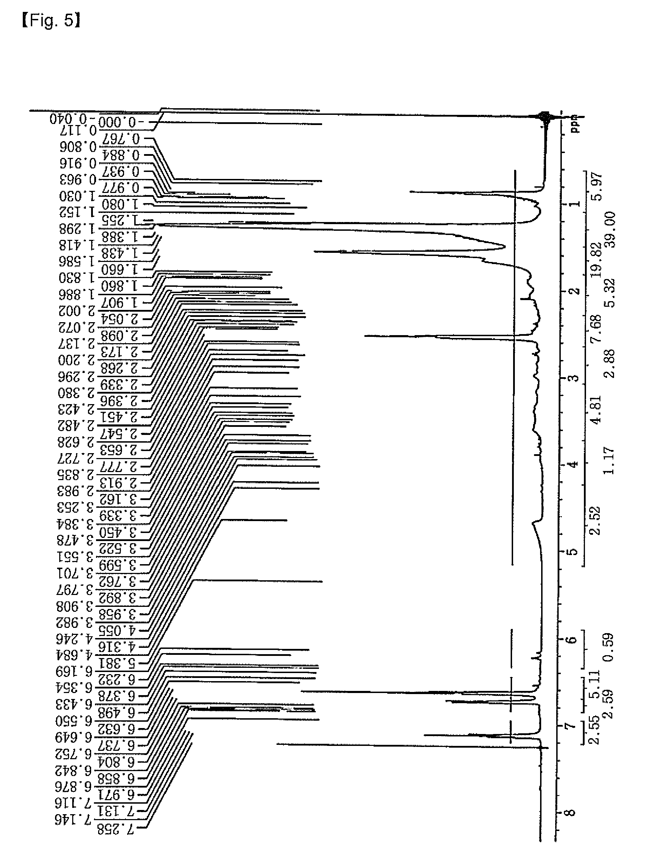

Fig. 4 and 5 illustrate the results of NMR analysis of cardanol and the cardanol-based organic vulcanizing agent, respectively.

### [Comparative Example 1] Preparation of Rubber Compound Composition

According to the composition of the following Table 2, 10 parts by weight of carbon black (N330), 70 parts by weight of silica (Z-175), 6.3 parts by weight of a silane coupling agent (Si-69), 3 parts by weight of zinc oxide (ZnO), 1.5 parts by weight of stearic acid, 5 parts by weight of anti-aging agent (6PPD) and 2.0 parts by weight of a dispersant (Wb222) are introduced to and mixed with 100 parts by weight of raw material rubber including 80 parts by weight of solution polymerized styrene butadiene rubber and 20 parts by weight of butadiene rubber, in a Banbury mixer. The reaction mixture is subjected to reaction at 140°C for 5 minutes to obtain a rubber compound. To the rubber compound, 2.1 parts by weight of Vulcanizing Agent-1 (inorganic sulfur) and 1.7 parts by weight of N-cyclohexyl-2-benzothiazol sulfonamide (CZ) as a vulcanization accelerator are added. Then, the mixture is compounded at 90°C to obtain a rubber compound containing a vulcanizing agent. After that, the rubber compound is subjected to vulcanization at 160°C for 15 minutes to obtain rubber. Herein, the silica has a DBP oil absorption of 191 ml/100 g, BET surface area of 161 m²/g, and a micropore volume of 0.018 cc/g. The carbon black has a DBP absorption of 101 ml/100 g, iodine absorption of 82 mg/g and a tint (% of IRB #3) of 104. In Table 2, Vulcanizing Agent-1 refers to inorganic sulfur generally used in the art.

### [Comparative Example 2] Preparation of Rubber Compound Composition

A rubber compound composition is prepared by using the same composition and process as Comparative Example 1, except that a commercially available active sulfur-releasing organic vulcanizing agent (Vulcanizing Agent 2) is used in combination with Vulcanizing Agent-1 (inorganic sulfur) of Comparative Example 1 according to the composition of Table 2.

### [Example 3] Preparation of Rubber Compound Composition

A rubber compound composition is prepared by using the same composition and process as Comparative Example 1, except that the novel cardanol-based organic vulcanizing agent obtained from Example 1 is used instead of a part of Vulcanizing Agent-1 of Comparative Example 1 according to the composition of Table 2.

### [Example 4] Preparation of Rubber Compound Composition

A rubber compound composition is prepared by using the same composition and process as Comparative Example 1, except that the novel cardanol-based organic vulcanizing agent obtained from Example 2 is used instead of a part of Vulcanizing Agent-1 of Comparative Example 1 according to the composition of Table 2.

**[Table 2]**

| Material Compounding Ratios of Rubber Compound Compositions | | | | |
|---|---|---|---|---|
| | Comp. Ex. 1 | Comp. Ex. 2 | Ex. 3 | Ex. 4 |
| Rubber (raw material) | 100 | 100 | 100 | 100 |
| Carbon black | 10 | 10 | 10 | 10 |
| Silica | 70 | 70 | 70 | 70 |
| Silane coupling agent | 6.3 | 6.3 | 6.3 | 6.3 |
| Zinc oxide | 3 | 3 | 3 | 3 |
| Stearic acid | 1.5 | 1.5 | 1.5 | 1.5 |
| Anti-aging agent | 5 | 5 | 5 | 5 |
| Dispersant | 2 | 2 | 2 | 2 |
| Vulcanizing Agent-1 (inorganic sulfur) | 2.1 | 1.5 | 1.5 | 1.5 |
| Vulcanizing Agent-2 (active sulfur-releasing organic vulcanizing agent (sulfur content 30%)) | 0 | 2 | 0 | 0 |
| Vulcanizing Agent-3 (caranol-based vulcanizing agent of Ex. 1 (sulfur content 30.1%)) | 0 | 0 | 2 | 0 |
| Vulcanizing Agent-4 (caranol-based vulcanizing agent of Ex. 2 (sulfur content 36.2%)) | 0 | 0 | 0 | 1.7 |
| Vulcanization accelerator | 1.7 | 1.7 | 1.7 | 1.7 |

### [Test Example 1] Analysis of Physical Properties of Rubber Compound Composition

To determine the physical properties of the novel cardanol-based organic vulcanizing agent according to the present invention, the physical properties of the rubber compound compositions according to Comparative Examples 1 and 2 and Examples 3 and 4 are tested. The results are shown in the following Table 3.

**[Table 3]**

| Physical Properties of Rubber Compound Compositions | | | | |
|---|---|---|---|---|
| | Comp. Ex. 1 | Comp. Ex. 2 | Ex. 3 | Ex. 4 |
| MN (Mooney Viscosity) | 80 | 83 | 79 | 77 |
| T40 (curing time) | 5.6 | 5.0 | 5.3 | 5.3 |
| Hardness | 69 | 68 | 68 | 69 |
| 300% modulus | 140 | 141 | 150 | 145 |
| Tensile strength (T/S) | 190 | 194 | 202 | 210 |
| William (%) (wear degree) | 1.38 (100) | 1.13 (122) | 1.02 (135) | 0.98(141) |
| FPS (expected mileage) | 45,776 (100) | 50,124 (109) | 65,832 (144) | 71,480 (156) |
| 0°C Tan δ (wet traction) | 0.7425 (100) | 0.787 (106) | 0.810 (109) | 0.825 (111) |
| 60°C Tan δ (rolling resistance) | 0.1098 (100) | 0.0996 (110) | 0.0964 (114) | 0.0959 (114) |

As can be seen from Table 3, the rubber compound compositions (Examples 3 and 4) containing the novel cardanol-based organic vulcanizing agent according to the present invention provides an improved tensile strength (T/S) and 0°C Tan δ (wet traction) as compared to Comparative Example 1. In addition, the index related with 60°C Tan δ (rolling resistance) is also improved significantly. Further, the novel organic vulcanizing agent shows significantly improved physical properties, particularly FPS (expected mileage), as compared to the commercially available organic vulcanizing agent (Comparative Example 2).

### [Test Example 2] Wear Test in Actual Car for Tires Using Rubber Compound Compositions

To determine the performance of tires using the novel cardanol-based organic vulcanizing agent according to the present invention, Comparative Examples 1 and 2 and Examples 3 and 4 are subjected to a wear test in an actual car. The results are shown in the following Table 4.

**[Table 4]**

| Wear Property Test of Tires in Actual Car | | | | | | | |
|---|---|---|---|---|---|---|---|
| | | Comp. Ex. 1 | Comp. Ex. 2 | Comp. Ex. 1 | Ex. 3 | Comp. Ex. 1 | Ex. 4 |
| 20,000km | Front wheel | 27,442 (100) | 30,119 (110) | 22,396 (100) | 24,636 (110) | 30,641 (100) | 38,678 (126) |
| | Rear wheel | 80,384 (100) | 84,736 (105) | 74,067 (100) | 79,992 (108) | 85,872 (100) | 111,365 (130) |

As can be seen from Table 4, the tires (Examples 3 and 4) using the novel cardanol-based organic vulcanizing agent according to the present invention show significantly improved wear property as compared to Comparative Example 1. The performance of Example 3 is similar to that of Comparative Example 2. Example 4 shows significantly improved wear property as compared to Comparative Examples 1 and 2 and Example 3. Thus, it can be seen that a higher content of diene in cardanol provides higher performance.

## Claims

1. A method for preparing a cardanol-based organic vulcanizing agent, comprising reacting cashew nut extract with powdered sulfur.

2. The method for preparing a cardanol-based organic vulcanizing agent according to claim 1, wherein the cashew nut extract is cardanol.

3. The method for preparing a cardanol-based organic vulcanizing agent according to claim 1, which includes reacting 100 parts by weight of cashew nut extract with 10-100 parts by weight of powdered sulfur.

4. The method for preparing a cardanol-based organic vulcanizing agent according to claim 1, wherein the reaction is carried out at 130-200°C.

5. The method for preparing a cardanol-based organic vulcanizing agent according to claim 1, which further comprises dissolving the product obtained by reacting cashew nut extract with powdered sulfur into a solvent to perform purification.

6. The method for preparing a cardanol-based organic vulcanizing agent according to claim 5, wherein the solvent is at least one selected from the group consisting of ethyl acetate, n-hexane and ether.

7. The method for preparing a cardanol-based organic vulcanizing agent according to claim 5, wherein the solvent is used in an amount of 150-200 parts by weight based on 100 parts by weight of the non-purified cardanol-based organic vulcanizing agent.

8. The method for preparing a cardanol-based organic vulcanizing agent according to claim 1, wherein the organic vulcanizing agent is represented by the following Chemical Formula 1 or 2: 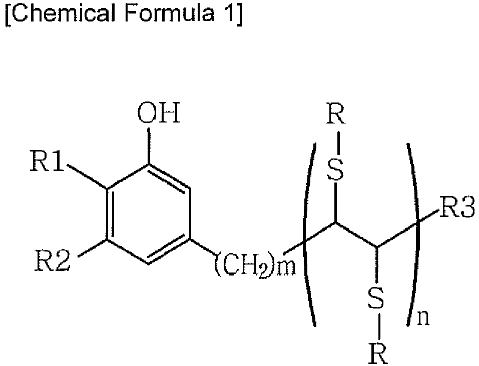 wherein m is an integer of 6 to 13, n is an integer of 1 or 2, R1 is H or methyl, R2 is H or a hydroxyl group, R3 is C_{n'}H_{2n'+1} (wherein n' is an integer of 2 to 5), and R groups are the same or different and each R represents H, Sₙ"H (wherein n" is an integer of 1 to 5) or -S group of 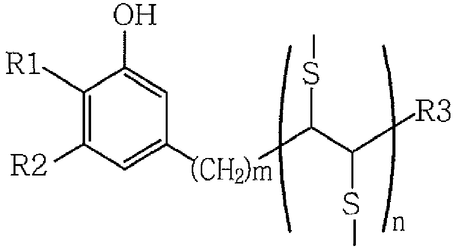 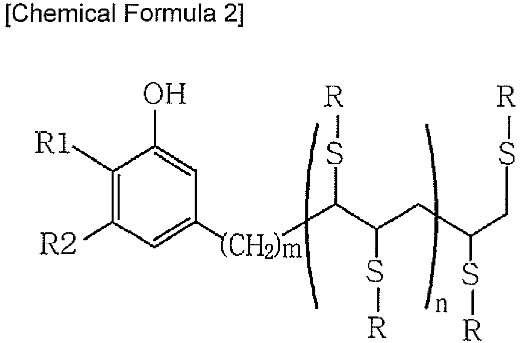 wherein m is an integer of 6 to 13, n is an integer of 1 or 2, R1 is H or methyl, R2 is H or a hydroxyl group, and R groups are the same or different and each R represents H, Sₙ"H (wherein n" is an integer of 1 to 5) or -S group of

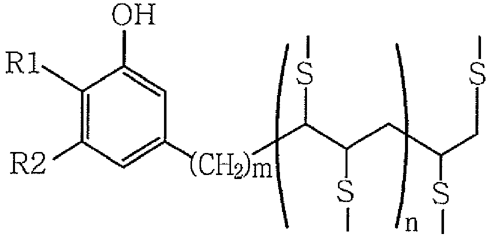

9. The method for preparing a cardanol-based organic vulcanizing agent according to claim 1, wherein the cardanol-based organic vulcanizing agent is represented by the following Chemical Formula 3, 4 or 5: 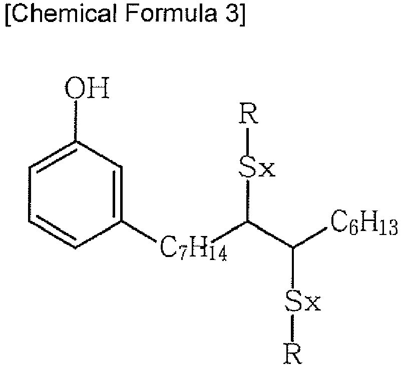 wherein x is an integer of 1 to 4, and R is H. 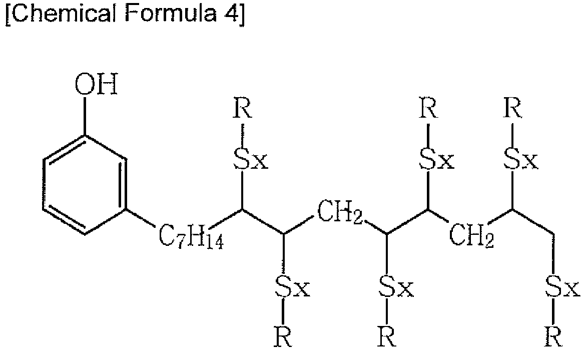 wherein x is an integer of 1 to 4, and R is H. 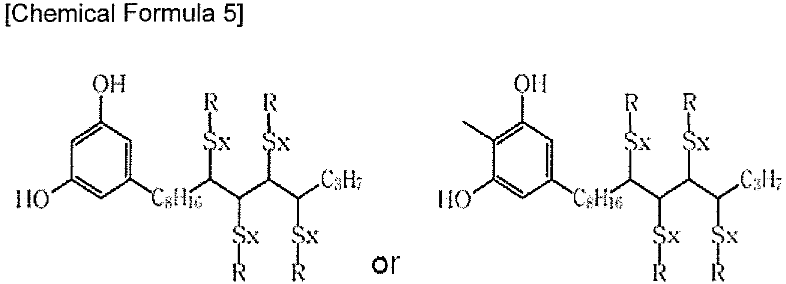 wherein x is an integer of 1 to 4, and R is H.

10. A cardanol-based organic vulcanizing agent obtained by the method as defined in any one of claims 1 to 9 and represented by the following Chemical Formula 1 or 2: 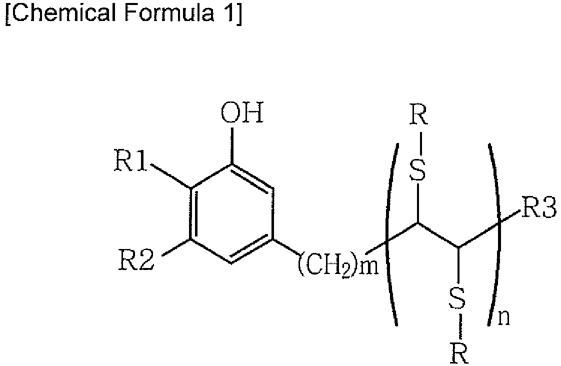 wherein m is an integer of 6 to 13, n is an integer of 1 or 2, R1 is H or methyl, R2 is H or a hydroxyl group, R3 is C_{n'}H_{2n'+1} (wherein n' is an integer of 2 to 5), and R groups are the same or different and each R represents H, S_{n"}H (wherein n" is an integer of 1 to 5) or-S group of 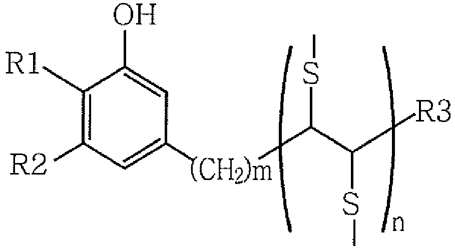 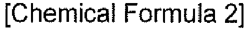 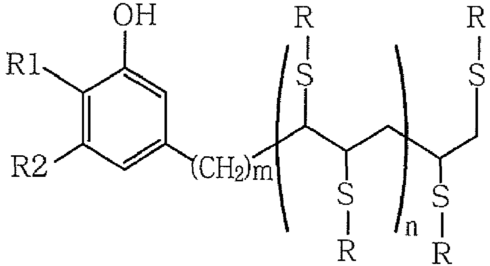 wherein m is an integer of 6 to 13, n is an integer of 1 or 2, R1 is H or methyl, R2 is H or a hydroxyl group, and R groups are the same or different and each R represents H, S_{n"}H (wherein n" is an integer of 1 to 5) or -S group of

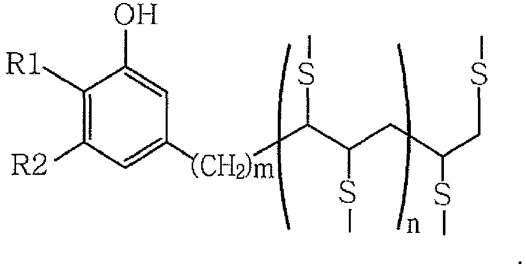

11. A rubber compound composition comprising the cardanol-based organic vulcanizing agent as defined in claim 10.

12. The rubber compound composition according to claim 11, which comprises 1-2 parts by weight of the cardanol-based organic vulcanizing agent, 50-70 parts by weight of silica and 10-30 parts by weight of carbon black based on 100 parts by weight of rubber as a raw material.

13. The rubber compound composition according to claim 11, which is for use in tire treads.
